# EUROPEAN PATENT SPECIFICATION

(11) **EP 2 258 355 B1**
(45) Date of publication and mention of the grant of the patent: **22.01.2020**
(21) Application number: 09715476.9
(22) Date of filing: 09.02.2009
(51) Int. Cl.: A61K 9/70, A61K 47/02, A61K 47/04, A61K 47/10, A61K 47/12, A61K 47/32, A61K 47/36

(54) **PATCH**
PFLASTER
PATCH

(30) Priority: 27.02.2008 JP 2008046804
(43) Date of publication of application: 08.12.2010
(73) Proprietor: Hisamitsu Pharmaceutical Co., Inc., Tosu-shi, Saga 841-0017 (JP)
(72) Inventor: UCHIDA, Naoyuki, Ibaraki 305-0856 (JP); FUJITA, Naoko, Ibaraki 305-0856 (JP)
(74) Representative: Grünecker Patent- und Rechtsanwälte PartG mbB
(86) International application number: PCT/JP2009/052181
(87) International publication number: WO 2009/107479

(56) References cited:
- EP-A1- 0 788 792
- WO-A1-96/39136
- WO-A1-2006/082728
- WO-A1-2007/129712
- WO-A2-01/43734
- JP-A- H09 299 444
- JP-A- 2007 016 020
- JP-T- 2002 509 874
- JP-T- 2002 509 878
- JP-T- 2002 509 879
- JP-T- 2005 528 413
- JP-T- 2005 535 686
- US-A1- 2003 104 041
- US-A1- 2004 220 262
- US-B1- 6 638 528
- US-B1- 6 899 894

## Description

### Technical Field

The present invention relates to a patch.

### Background Art

A wide variety of drug-containing patches are developed in the expectations of reducing adverse reaction due to the avoidance of gastrointestinal tissue absorption of the drug and first-time passage to the liver and the improvement of patient compliance.

However, not all the drugs exhibit good tissue absorption in transdermal administration, transmembrane administration, transnail administration, or the like, and thus various studies have been conducted to enhance the tissue absorption of the drugs.

The drugs are distributed in the form of acid addition salt in the market from viewpoints of the handleability and stability thereof. However, when a drug acid addition salt is directly applied to the transdermal administration, or the like, it is generally known that the tissue absorption tends to be decreased. On the other hand, it is also known that the free base (free form) of a drug is preferable in view of the tissue absorption.

Accordingly, techniques wherein an acid addition salt of a drug used for a patch is neutralized (desalted) using a metal hydroxide such as sodium hydroxide, which is a strong base to completely desalt the acid addition salt have been studied (e.g., Patent Documents 1 and 2), but all of these techniques are those in which the formed metal salt is either removed by carrying out filtration beforehand or mixed with a pressure-sensitive adhesive base without further treatment.
Patent Document 1: Japanese Unexamined Patent Application Publication No. 2007-16020
Patent Document 2: Japanese Unexamined Patent Application Publication No. 2002-509874

WO 96/39136 discloses transdermal formulations comprising ropinirole in a saline/propylene glycol vehicle for use in the treatment of Parkinson's Disease.

US 6,638,528 B1 relates to a transdermal drug delivery system wherein the use of a polymeric plastic material provides a release rate regulating effect on the active agents incorporated into the adhesive matrix composition. Optionally, a crystallization inhibitor or solubility enhancer may also be employed in the transdermal drug delivery system.

US 6,899,894 B1 relates to a transdermal therapeutic system in plaster form for controlled release of oestradiol in combination with norethisterone acetate, comprising a backing layer, a reservoir supersaturated with active ingredients which as attached to said backing layer and prepared using polyacrylate pressure-sensitive adhesives and crystallization inhibitors, and a detachable protective layer, which is characterized in that the crystallization inhibitor is an amino-containing polymer.

US 2003/0104041 A1 discloses a method for enhancing the flux of an analgesic agent through a body surface which includes administering a basic permeation enhancer comprising a pharmaceutically acceptable inorganic base.

### Disclosure of the Invention

### Problem to be Solved by the Invention

However, the present inventors found that when an easier production process wherein the free form of a drug and a metal salt (sodium chloride, or the like) are formed by the neutralization reaction of an acid addition salt of the drug and the metal salt is mixed with a pressure-sensitive adhesive base without being removed by filtration is employed, the metal salt formed by the neutralization reaction sometimes precipitates in the form of crystal, which aggregates and grows over time adversely affecting the production efficiency, product stability and product properties of a patch.

Thus, an object of the present invention is to provide a patch with suppressed over-time aggregation and growth of the metal salt formed when a drug salt is neutralized.

### Means for Solving Problem

The present inventors conducted extensive studies to accomplish the above object and found that a salt derived from a drug acid addition salt formed after a neutralization reaction has a possibility to aggregate and grow around a small amount of the residue of a polar solvent (water, methanol, ethanol, or the like) used when a patch is produced or the neutralization reaction is carried out, and further found that the aggregation and growth can be suppressed by containing an adsorbent in a pressure-sensitive adhesive layer.

More specifically, the present invention is to provide a patch comprising a pressure-sensitive adhesive layer, a backing layer and a release sheet, the pressure-sensitive adhesive layer being laminated on the backing layer and the release sheet being attached on the pressure-sensitive adhesive layer, wherein said pressure sensitive adhesive layer comprises a basic drug, a metal salt, an adsorbent and a pressure-sensitive adhesive base, wherein:
the basic drug in its free base form and the metal salt being obtainable by a neutralization reaction between the drug acid addition salt and a neutralizer which is added in an amount to convert the basic drug as whole or a part to the state of the free basic drug,
the basic drug is present in the pressure sensitive adhesive layer as its free base form by neutralization or both as its free base form and as its acid addition salt form due to incomplete neutralization,
the metal salt is at least one metal salt selected from the group consisting of sodium chloride, magnesium chloride, potassium chloride, sodium citrate, sodium tartrate, sodium bromide and sodium succinate, and
the content of the metal salt is the same or less number of moles of the acid in the basic drug acid addition salt;
the adsorbent is an adsorbent that adsorbs a polar solvent comprised in the patch wherein the adsorbent is at least one adsorbent selected from the group consisting of talc, kaoline, bentonite, hydrous silica, fumed silica, aminoalkyl methacrylate copolymer, crospovidone, carboxy vinyl polymer, zinc oxide, dextrin and dried aluminum hydroxide gel;
the pressure-sensitive adhesive base is selected from acrylic pressure-sensitive adhesive bases, rubber pressure-sensitive adhesive bases and silicone pressure-sensitive adhesive bases; and
wherein the basic drug acid addition salt is selected from any one from the group of flurazepam hydrochloride, rilmazafone hydrochloride, medetomidine hydrochloride and dexmedetomidine hydrochloride, butorphanol tartrate, perisoxal citrate, methamphetamine hydrochloride, methylphenidate hydrochloride, imipramine hydrochloride, sertraline hydrochloride, paroxetine hydrochloride, citalopram hydrobromide, fluoxetine hydrochloride, chlorpromazine hydrochloride, lidocaine hydrochloride, procaine hydrochloride, tetracaine hydrochloride, dibucaine hydrochloride, propitocaine hydrochloride, propiverine hydrochloride, solifenacin succinate, tizanidine hydrochloride, eperisone hydrochloride, ritodrine hydrochloride, meluadrine tartrate, trihexyphenidyl hydrochloride, amantadine hydrochloride, talipexole hydrochloride, selegiline hydrochloride, loberine hydrochloride, naloxone hydrochloride, ergotamine tartrate, flunarizine hydrochloride, cyproheptadine hydrochloride, diphenylpyraline hydrochloride, tulobuterol hydrochloride, procaterol hydrochloride, clenbuterol hydrochloride, fenoterol hydrobromide, isoprenaline hydrochloride, dopamine hydrochloride, diltiazem hydrochloride, verapamil hydrochloride, nicametate citrate, tolazoline hydrochloride; varenicline tartrate, flunarizine hydrochloride, nicardipine hydrochloride, manidipine hydrochloride, benidipine hydrochloride, temocapril hydrochloride, imidapril hydrochloride, metoprolol tartrate, betaxolol hydrochloride, arotinolol hydrochloride, celiprolol hydrochloride, carteolol hydrochloride, bevantolol hydrochloride, clonidine hydrochloride, propranolol hydrochloride, alprenolol hydrochloride, procainamide hydrochloride, mexitilene hydrochloride, procarbazine hydrochloride, irinotecan hydrochloride, buformin hydrochloride, cetraxate hydrochloride, azelastine hydrochloride, difenidol hydrochloride, bacampicillin hydrochloride, ticlopidine hydrochloride, galantamine hydrobromide, ondansetron hydrochloride, granisetron hydrochloride, ramosetron hydrochloride, azasetron hydrochloride, morphine hydrochloride, cocaine hydrochloride, pethidine hydrochloride, terbinafine hydrochloride, butenafine hydrochloride, amorolfine hydrochloride, and neticonazole hydrochloride,
wherein the neutralizer is sodium hydroxide, potassium hydroxide or magnesium hydroxide.

With reference to an example of a drug being a basic drug, the metal salt contained in the patch of the present invention is now described. When expressing a basic drug as "A", a substance capable of forming a drug acid addition salt by bonding to the drug "A" as "HX" and a drug acid addition salt as "A·HX", the neutralization reaction mentioned above can be expressed as A·HX + MOH → A+MX+H₂O and the salt formed as a result of the neutralization reaction is expressed as "MX". The metal salt in the patch of the present invention is the "MX" in this example and contains the component "X" of the substance "HX" capable of forming a drug salt by bonding to the drug "A". Further, the content of the "MX" is not greater than the number of moles of the "HX" in the "A·HX".

The adsorbent contained in the patch of the present invention is the one which adsorbs polar solvent such as the water produced by the above neutralization reaction and remaining in the patch, the water used in the production process and remaining in the patch, methanol and ethanol and consists of an inorganic and/or organic substance.

In the present invention, since the kind and content of a metal salt are limited as above and an adsorbent for adsorbing a polar solvent is also contained, it is considered that the over-time aggregation and growth of a metal salt formed when a patch is produced by neutralizing a drug salt are suppressed, thereby enhancing the production efficiency, product stability and product properties of the patch.

The metal salt contained in the patch of the present invention may be those formed during the production as described above, but may also be those formed in the patch after the production (i.e., during storage after the production and before use).

In the present invention, the drug is a basic drug, and the metal salt is at least one selected from the group consisting of metal chlorides, metal bromides, and organic acid metal salts. A particularly preferable metal salt is at least one selected from the group consisting of sodium chloride, magnesium chloride, potassium chloride, sodium citrate, sodium tartrate, sodium bromide and sodium succinate.

The adsorbent is at least one adsorbent selected from the group consisting of talc, kaoline, bentonite, hydrous silica, fumed silica, amino alkyl methacrylate copolymers, crospovidone, carboxy vinyl polymers, zinc oxide, dextrin and dried aluminum hydroxide gel, owing to a high adsorbability of a polar solvent contained in the patch.

Since the effects of the present invention are significantly achieved and a wide variety of potential ingredients are applicable, it is preferred that the basic drug be the one generated from a basic drug acid addition salt and that the basic drug acid addition salt be hydrochloride, citrate, tartrate or succinate of a basic drug.

The patch of the present invention may be applied directly to the skin as a so-called plaster, or may be applied as a plaster agent by forming an pressure-sensitive adhesive layer on a backing layer and containing the patch in the pressure-sensitive adhesive layer.

### Effect of the Invention

According to the present invention, a patch in which the over-time aggregation and growth of a metal salt formed even when a drug salt is neutralized are suppressed is provided.

### Brief Description of the Drawings

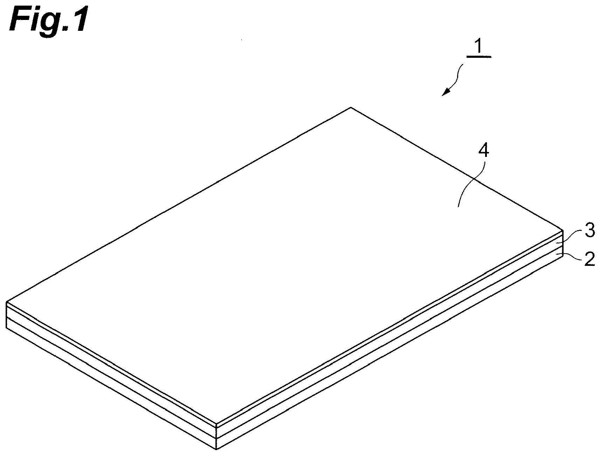

Fig. 1 is a perspective view showing a preferable embodiment of a patch of the present invention.

### Description of Symbols

1. Patch, 2. Backing layer, 3. Pressure-sensitive adhesive layer, 4. Release sheet

### Best Modes for Carrying Out the Invention

Hereinafter, preferable embodiments are described in detail with reference to the drawing. A part of the drawing is depicted in a larger manner for easier understanding, and the size ratio does not necessarily correspond with that of the description.

Fig. 1 is a perspective view showing a preferable embodiment of the patch of the present invention. In Fig. 1, a patch 1 is provided with a backing layer 2, a pressure-sensitive adhesive layer 3 laminated on the backing layer 2, and a release sheet 4 attached on the pressure-sensitive adhesive layer 3. The pressure-sensitive adhesive layer 3 contains a drug, a metal salt and an adsorbent. Further, the metal salt contains a substance capable of forming a drug salt by bonding to the drug or a component thereof, and the content of the metal salt is not greater than the number of moles of the substance capable of forming a drug salt by bonding to the drug or a component thereof when a drug salt is formed in the same mole as the drug contained in the patch.

The pressure-sensitive adhesive layer 3 may be laminated to two or more layers, and may be laminated not only on one side but also on both sides of the backing layer 2. When applying the patch, the release sheet 4 is removed before use.

The material for the backing layer 2 is not limited insofar as it is typically usable for patches, and elastic or non-elastic materials can be used. Specific examples of the preferably used material include films or sheets formed with a synthetic resin such as polyethylene terephthalate, polyethylene, polypropylene, polybutadiene, ethylene vinyl acetate polymer, polyvinyl chloride, polyester, nylon, and polyurethane, or laminates thereof, porous membranes, foams, fabrics and nonwoven fabrics and paper materials.

The pressure-sensitive adhesive layer 3 contains a pressure-sensitive adhesive base. The pressure-sensitive adhesive base is not limited insofar as it can be a base for the pressure-sensitive adhesive layer 3, and examples include acrylic pressure-sensitive adhesive bases, rubber pressure-sensitive adhesive bases, and silicone pressure-sensitive adhesive bases.

Examples of the preferably usable acrylic pressure-sensitive adhesive base include homopolymer or copolymer of (meth)acrylate, and copolymer of alkyl (meth)acrylate ester and other functional monomers. (Meth)acryl means acryl or methacryl.

Examples of the preferably used rubber pressure-sensitive adhesive base include natural rubbers, synthetic rubbers, styreneisoprene-styrene block copolymer (hereinafter abbreviated as "SIS"), isoprene rubber, polyisobutylene (hereinafter abbreviated as "PIB"), styrene-butadiene-styrene block copolymer (hereinafter abbreviated as "SBS"), styrene-butadiene rubber (hereinafter abbreviated as "SBR") and polybutene, and the rubber pressure-sensitive adhesive base is typically used by adding a tackifier.

Usable silicone pressure-sensitive adhesive base are those containing polydimethyl siloxane or the like as a main component, and these are typically used by adding a tackifier such as MQ resin.

Among the pressure-sensitive adhesive bases listed above, an acrylic ester copolymer or SIS to which a tackifier is added is particularly preferably used.

These pressure-sensitive adhesive bases may be used singly, or two or more may be used in combination. The amount of a pressure-sensitive adhesive base to be added is preferably 10 to 95% by mass, more preferably 15 to 80% by mass, particularly preferably 20 to 70% by mass, on the basis of the total mass of the pressure-sensitive adhesive layer 3 when considering the formation of the pressure-sensitive adhesive layer 3 and the tissue permeability of the active ingredients.

The pressure-sensitive adhesive layer 3 contains a drug. The drug includes a drug salt which has become a free form by a neutralization reaction and a drug salt remained in the form of salt due to an incomplete neutralization.

The drugs can be used in combination of two or more as necessary if no inconvenience is caused by the interaction. Further, considering that the sufficient potency as a patch is attained, the product properties and tissue absorption, the drug is added in a proportion of preferably 0.5 to 50% by mass, particularly preferably 1 to 30% by mass, on the basis of the total mass of the pressure-sensitive adhesive layer 3.

The drug is a basic drug generated from an acid addition salt. Examples of the acid addition salt form include hydrochloride, oxalate, citrate, hydroiodide, hydrobromate, tartrate, and succinate.

The pressure-sensitive adhesive layer 3 contains a metal salt containing a substance capable of forming a drug salt by bonding to the drug or a component thereof. Such a metal salt is preferably those formed during production steps. Since the drug is a basic drug generated from an acid addition salt, it is desirable to use an acid addition salt of a basic drug as a starting material, cause a neutralization reaction of the drug by mixing the salt with a neutralizer, allow the free form of the drug having a higher tissue absorption to be present in the pressure-sensitive adhesive layer 3 and cause the metal salt produced as a result of the neutralization reaction of the drug to be also contained therein.

The type of the metal salt produced as a result of the neutralization reaction (desalting reaction) is determined by a drug salt and a neutralizer for neutralizing the drug salt. The metal salt produced when neutralizing an acid addition salt of a basic drug is at least one of sodium chloride, magnesium chloride, potassium chloride, sodium citrate, sodium tartrate, sodium bromide and sodium succinate.

The neutralizer used for the neutralization reaction is not limited, but when a basic drug produced from an acid addition salt is used, a strong base is suitable for completely desalting an acid addition salt of a basic drug, with a hydroxide of an alkali metal is particularly preferably used. Specific examples of the neutralizer include sodium hydroxide, potassium hydroxide, and magnesium hydroxide, with sodium hydroxide being particularly preferable among these. The neutralizer is added to convert a basic drug as a whole or a part to the state of free base (free form). To obviate the decomposition of the drug caused by an excessive amount of a neutralizer at this step, the neutralizer is preferably added within a range from 0.5 to 4 equivalent amounts to the equivalent amount of the drug acid base. The addition may be carried out once during the production steps, or carried out several times in a divided manner.

The patch 1 produced via a neutralization reaction contains a metal salt formed as a result of the neutralization in the pressure-sensitive adhesive layer 3.

The metal salt present in the pressure-sensitive adhesive layer 3 is likely to aggregate and grow around a small amount of the residue of a polar solvent (water, methanol, ethanol, etc.) used during the production of the patch and the neutralization reaction, but the aggregation and growth of the metal salt can be suppressed by allowing the pressure-sensitive adhesive layer 3 to contain an adsorbent. Further, the crystal can be dispersed uniformly by containing an adsorbent. Owing to this, the production efficiency, product stability and product properties of the patch 1 are enhanced.

Thus, the pressure-sensitive adhesive layer 3 contains an adsorbent, which may be one of the inorganic substances and organic substances described as having hygroscopic properties, dampproofing properties, adsorptive properties among the additives listed in "Japanese Pharmaceutical Excipients Directory 2000, published on April 28, 2000, 1st edition" and aminoalkyl methacrylate copolymers and zinc oxide, which are not described in the above "Japanese Pharmaceutical Excipients Directory 2000" but have been known to have adsorptive properties. Among these, at least one adsorbent is selected from talc, kaoline, and bentonite; fumed silica and hydrous silica; zinc oxide and dried aluminum hydroxide gel; dextrin, aminoalkyl methacrylate copolymers, crospovidone, and carboxy vinyl polymers. These adsorbents may be used in combination of two or more as necessary.

The content of the adsorbent in the pressure-sensitive adhesive layer 3 is preferably 0.5 to 50% by mass on the total mass basis of the pressure-sensitive adhesive layer 3. A content of 0.5% by mass or lower tends not to achieve sufficient effects to control the aggregation and growth of the metal salt crystal or to disperse the crystal uniformly. Conversely, a content of 50% by mass or higher has a tendency of reducing the adhesion of the pressure-sensitive adhesive layer 3, likely making it difficult to apply the patch.

The patch 1 of the present invention may contain as necessary, in addition to the above compositions, tackifier, plasticizer, absorption enhancer, antioxidant, filler, crosslinking agent, preservative and ultraviolet absorber.

Examples of the usable tackifier include rosin resins such as "Ester Gum (tradename, Arakawa Chemical Industries, Ltd.)", "Hariester (tradename, Harima Chemicals, Inc.)", "Pentalyn (tradename, Eastman Chemical Company)", "Foral (tradename, Eastman Chemical Company)", terpene resins such as "YS resin (tradename, Yasuhara Chemical Co., Ltd.)", "Piccolyte (tradename, Loos and Dilworth)", petroleum resins such as "Arkon (tradename, Arakawa Chemical Industries, Ltd.)", "Regalrez (tradename, Eastman Chemical Company)", "Piccolastic (tradename, Eastman Chemical Company)", "Escorez (tradename, ExxonMobil Chemical Company)", "Wingtack (tradename, Goodyear)", "Quintone (tradename, Zeon Corporation)", phenol resins, and xylene resins.

These tackifiers may be used singly or in combination of two or more. The amount of the tackifier to be added is preferably 10 to 90% by mass, more preferably 15 to 70% by mass, particularly preferably 20 to 60% by mass, on the basis of the total mass of the pressure-sensitive adhesive layer 3, when considering the sufficient adhesion of and the local irritation when removing the patch 1.

Examples of the plasticizer include petroleum oils such as paraffin process oils, naphthene process oils, and aromatic process oils; squalane, squalene; plant oils such as olive oil, camellia oil, castor oil, tall oil, and peanut oil; dibasic esters such as dibutyl phthalate, and dioctyl phthalate; liquid rubbers such as polybutene and liquid isoprene rubber; and diethylene glycol, polyethylene glycol, propylene glycol, and dipropylene glycol. These plasticizers can be used singly or two or more can be used in combination.

In the embodiment of the present invention, the liquid paraffin and liquid polybutene are preferably used.

The content of the above plasticizer in the pressure-sensitive adhesive layer 3 is preferably 1 to 60% by mass, more preferably 2 to 50% by mass, particularly preferably 3 to 40% by mass, on the basis of the total mass of the pressure-sensitive adhesive layer 3, considering the maintenance of the sufficient adhesion of the patch 1.

Examples of the preferably used absorption enhancer include fatty alcohols such as isostearyl alcohol, fatty acids such as capric acid, fatty acid derivatives such as propyleneglycol monolaurate and isopropyl myristate, propylene glycol, polyethylene glycol, and lauric acid diethanolamine. These absorption enhancers may be used singly, or in combination of two or more. The content of the absorption enhancer is preferably 1 to 30% by mass, more preferably 3 to 20% by mass, particularly preferably 5 to 15% by mass, on the basis of the total mass of the patch 1, considering the sufficient permeability of the active ingredients to the tissue, the local irritation, and the like, as a patch.

Examples of the usable antioxidant include tocopherols and ester derivatives thereof, ascorbic acid, ascorbyl stearate, nordihydroguaiaretic acid, dibutylhydroxytoluene (hereinafter abbreviated as BHT), and butylated hydroxyanisole, with BHT being particularly preferably used.

Examples of the filler include aluminum hydroxide, calcium carbonate, magnesium carbonate; silicates such as aluminum silicate and magnesium silicate; silicic acid, barium sulfate, calcium sulfate, calcium zincate, zinc oxide, and titanium oxide.

Examples of the crosslinking agent include thermosetting resins such as amino resin, phenol resin, epoxy resin, alkyd resin, and unsaturated polyester, isocyanate compounds, blocked isocyanate compounds, organic crosslinking agents as well as inorganic crosslinking agents such as metals and metal compounds.

Examples of the preservative preferably usable include disodium edetate, tetrasodium edetate, ethyl parahydroxybenzoate, propyl parahydroxybenzoate, and butyl parahydroxybenzoate.

Examples of the ultraviolet absorber include p-aminobenzoic acid derivatives, anthranilic acid derivatives, salicylic acid derivatives, coumarin derivatives, amino acid compounds, imidazoline derivatives, pyrimidine derivatives, and dioxane derivatives.

The above mentioned antioxidants, fillers, crosslinking agents, preservatives and ultraviolet absorbers can be added in a total amount of preferably 5% by mass or less, more preferably 3% by mass or less, particularly preferably 1% by mass or less, on the basis of the total mass of the pressure-sensitive adhesive layer 3.

Next, an embodiment of the production process of the patch 1 will be described.

First, a mixture for forming a pressure-sensitive adhesive layer 3 is prepared. Using a mixer, the above mentioned pressure-sensitive adhesive base, an acid addition salt of a drug, neutralizer, adsorbent and other ingredients are dissolved or dispersed in a solvent of the pressure-sensitive adhesive base to obtain a mixture for forming the pressure-sensitive adhesive layer 3.

Examples of the usable solvent for the pressure-sensitive adhesive base include toluene, hexane, ethyl acetate, cyclohexane, heptane, butyl acetate, ethanol, methanol, xylene, and isopropanol. These are selected as necessary in accordance with the ingredients to be dissolved or dispersed, and may be used singly or in combination of two or more.

Subsequently, the obtained mixture for forming the pressure-sensitive adhesive layer 3 is spread directly on a backing layer 2 to form the pressure-sensitive adhesive layer 3, or the mixture is spread on a release-treated paper or film to form the pressure-sensitive adhesive layer 3 and the backing layer 2 is placed thereon, followed by the press-bonding transfer of the pressure-sensitive adhesive layer 3 to the backing layer 2. Next, a release sheet 4 for protecting the pressure-sensitive adhesive layer 3 is adhered on the pressure-sensitive adhesive layer 3 to obtain the patch 1.

### Examples

Hereinafter, the present invention will be specifically described with reference to Examples, but is not limited thereto.

### Reference Example 1

Using a mixer, ropinirole hydrochloride, sodium hydroxide, Aerosil (registered trademark), liquid paraffin and toluene (solvent) were mixed in advance, and a separately prepared mixed solution of SIS (JSR Corporation, SIS5000), a terpene resin (YS resin) and toluene was added thereto and mixed to obtain a pressure-sensitive adhesive solution. The solution was spread on a release-treated film to dry and remove the solvent, thereby forming a pressure-sensitive adhesive layer. A backing layer was placed on the layer and the pressure-sensitive adhesive layer was subjected to press-bonding transfer to obtain a patch.

The content of the ingredients other than the solvent was ropinirole hydrochloride 15 parts by mass, sodium hydroxide 4 parts by mass (2.0 times equivalent to ropinirole hydrochloride), Aerosil (registered trademark) 5 parts by mass, liquid paraffin 24.1 parts by mass, SIS 20.1 parts by mass, and the terpene resin (YS resin) 31.8 parts by mass.

### Reference Examples 2 to 8

Patches of Reference Examples 2 to 8 were produced respectively in the same manner as in Example 1 except that aminoalkyl methacrylate copolymer, crospovidone, lactic acid, talc, dextrin, propylene glycol or polyvinylpyrrolidone was used in place of Aerosil (registered trademark) as an adsorbent in the same parts by mass (Table 1).

### Comparative Example 1

A patch of Comparative Example 1 was produced in the same manner as in Example 1 except that an adsorbent was not contained (Table 1). The mixing ratio of ropinirole hydrochloride, sodium hydroxide, liquid paraffin, SIS and the terpene resin (YS resin) was the same as in Example 1.

### Comparative Examples 2 to 20

Patches of Comparative Examples 2 to 21 were produced respectively in the same manner as in Example 1 except that isostearic acid, oleic acid, sorbitan monolaurate, sorbitan monooleate, isopropyl myristate, isopropyl palmitate, hexyl laurate, glycerol monooleate, triacetin, lauryl alcohol, myristyl alcohol, oleyl alcohol, polybutene, propylene glycol monolaurate, Tween 80 (registered trademark), lauric acid diethanolamide, isostearyl alcohol, octyldodecanol or benzyl alcohol was used in place of Aerosil (registered trademark) as an absorbent in the same parts by mass (Table 1).

### Example 9

After mixing terbinafine hydrochloride, sodium hydroxide, Aerosil (registered trademark), isopropyl myristate and ethyl acetate (solvent) in advance using a mixer, an acrylic pressure-sensitive adhesive (National Starch and Chemical Company, Duro-TAK87-2516, solid content: 41.5%) was added to and mixed with the mixture to obtain a pressure-sensitive adhesive solution. The solution was spread on a release-treated film to dry and remove the solvent, thereby forming a pressure-sensitive adhesive layer. A backing layer was placed on the layer and the pressure-sensitive adhesive layer was subjected to press-bonding transfer to obtain a patch.

The content of the ingredients other than the solvent was terbinafine hydrochloride 10 parts by mass, sodium hydroxide 2.4 parts by mass (2.0 times equivalent to terbinafine hydrochloride), Aerosil (registered trademark) 5 parts by mass, isopropyl myristate 10 parts by mass and acrylic pressure-sensitive adhesive base in the acrylic pressure-sensitive adhesive 72.6 parts by mass.

### Examples 10 to 18, 20 to 22 and Reference Example 19

Patches of Examples 10 to 18, 20 to 22 and Reference Example 19 were produced respectively in the same manner as in Example 9 except that aminoalkyl methacrylate copolymer, crospovidone, zinc oxide, bentonite, kaoline, polyvinylpyrrolidone, talc, hydrous silica or dried aluminum hydroxide gel was used in place of Aerosil (registered trademark) as an adsorbent in the same parts by mass (Table 2).

### Comparative Example 21

A patch of Comparative Example 21 was produced in the same manner as in Example 9 except that an adsorbent was not contained (Table 2). The mixing ratio of terbinafine hydrochloride, sodium hydroxide, Aerosil (registered trademark), isopropyl myristate and the acrylic pressure-sensitive adhesive base was the same as in Example 9.

### Example 23

After mixing varenicline tartrate, sodium hydroxide, crospovidone and methanol (solvent) in advance using a mixer, an acrylic pressure-sensitive adhesive material (National Starch and Chemical Company, Duro-TAK87-2516, solid content: 41.5%) was added to and mixed with the mixture to obtain a pressure-sensitive adhesive solution. The solution was spread on a release-treated film to dry and remove the solvent, thereby forming a pressure-sensitive adhesive layer. A backing layer was placed thereon and the pressure-sensitive adhesive layer was subjected to press-bonding transfer to obtain a patch.

The content of the ingredients other than the solvent was varenicline tartrate 10 parts by mass, sodium hydroxide 2.2 parts by mass (2.0 times equivalent to terbinafine hydrochloride), crospovidone 3 parts by mass and acrylic pressure-sensitive adhesive base in the acrylic pressure-sensitive adhesive 84.8 parts by mass.

### Example 24 and Reference Example 25

Patches of Example 24 and Reference Example 25 were produced respectively in the same manner as in Example 23 except that aminoalkyl methacrylate copolymer or polyvinylpyrrolidone was used in place of crospovidone as an adsorbent (Table 3).

### Comparative Example 22

A patch of Comparative Example 22 was produced in the same manner as in Example 23 except that an adsorbent was not contained (Table 3). The mixing ratio of varenicline tartrate and sodium hydroxide was made same as in Example 23, with the acrylic pressure-sensitive adhesive base being 87.8 parts by mass.

### Patch evaluation

The appearances of the patches immediately after production were examined and evaluated for the presence of aggregates of the metal salt crystal with naked eyes. Tables 1 to 3 show the adsorbents, content thereof and the evaluation results. The patches wherein no aggregates were found are indicated as "a", those wherein small aggregates of an average particle size of 100 to 250 µm were found but practically having no problem are indicated as "b", and those wherein many large aggregates of an average particle size of exceeding 250 µm are indicated as "c".

**Table 1**

| | Adsorbent | Content [parts by mass] | Result |
|---|---|---|---|
| Reference Example 1 | Aerosil (registered trademark) | 5 | a |
| Reference Example 2 | Aminoalkyl methacrylate copolymer | 5 | a |
| Reference Example 3 | Crospovidone | 5 | a |
| Reference Example 4 | Lactic acid | 5 | a |
| Reference Example 5 | Talc | 5 | a |
| Reference Example 6 | Dextrin | 5 | b |
| Reference Example 7 | Propylene glycol | 5 | b |
| Reference Example 8 | Polyvinylpyrrolidone | 5 | b |
| Comparative Example 1 | None | 0 | c |
| Comparative Example 2 | Isostearic acid | 5 | c |
| Comparative Example 3 | Oleic acid | 5 | c |
| Comparative Example 4 | Sorbitan monolaurate | 5 | c |
| Comparative Example 5 | Sorbitan monooleate | 5 | c |
| Comparative Example 6 | Isopropyl myristate | 5 | c |
| Comparative Example 7 | Isopropyl palmitate | 5 | c |
| Comparative Example 8 | Hexyl laurate | 5 | c |
| Comparative Example 9 | Glycerol monooleate | 5 | c |
| Comparative Example 10 | Triacetin | 5 | c |
| Comparative Example 11 | Lauryl alcohol | 5 | c |
| Comparative Example 12 | myristyl alcohol | 5 | c |
| Comparative Example 13 | Oleyl alcohol | 5 | c |
| Comparative Example 14 | Polybutene | 5 | c |
| Comparative Example 15 | Propylene glycol monolaurate | 5 | c |
| Comparative Example 16 | Tween 80 (registered trademark) | 5 | c |
| Comparative Example 17 | Lauric acid diethanolamide | 5 | c |
| Comparative Example 18 | Isostearyl alcohol | 5 | c |
| Comparative Example 19 | Octyldodecanol | 5 | c |
| Comparative Example 20 | Benzyl alcohol | 5 | c |

**Table 2**

| | Adsorbent | Content [parts by mass] | Result |
|---|---|---|---|
| Example 9 | Aerosil (registered trademark) | 5 | a |
| Example 10 | Aminoacryl methacrylate copolymer | 10 | a |
| Example 11 | Aminoacryl methacrylate copolymer | 15 | a |
| Example 12 | Crospovidone | 10 | a |
| Example 13 | Crospovidone | 15 | a |
| Example 14 | Zinc oxide | 3 | a |
| Example 15 | Zinc oxide | 5 | a |
| Example 16 | Zinc oxide | 7 | a |
| Example 17 | Bentonite | 5 | b |
| Example 18 | Kaoline | 5 | b |
| Reference Example 19 | Polyvinylpyrrolidone | 5 | b |
| Example 20 | Talc | 5 | b |
| Example 21 | Hydrous silica | 5 | b |
| Example 22 | Dried aluminum hydroxide gel | 5 | b |
| Comparative Example 21 | None | 0 | c |

**Table 3**

| | Adsorbent | Content [parts by mass] | Result |
|---|---|---|---|
| Example 23 | Crospovidone | 3 | a |
| Example 24 | Aminoacryl methacrylate copolymer | 3 | a |
| Reference Example 25 | Polyvinylpyrrolidone | 3 | b |
| Comparative Example 22 | None | 0 | c |

### Industrial Applicability

According to the present invention, a patch in which the over-time aggregation and growth of a metal salt formed even when a drug salt is neutralized are suppressed is provided.

## Claims

1. A patch comprising a pressure-sensitive adhesive layer, a backing layer and a release sheet, the pressure-sensitive adhesive layer being laminated on the backing layer and the release sheet being attached on the pressure-sensitive adhesive layer, wherein said pressure sensitive adhesive layer comprises a basic drug, a metal salt, an adsorbent and a pressure-sensitive adhesive base, wherein:
the basic drug in its free base form and the metal salt being obtainable by a neutralization reaction between the drug acid addition salt and a neutralizer which is added in an amount to convert the basic drug as whole or a part to the state of the free basic drug,
the basic drug is present in the pressure sensitive adhesive layer as its free base form by neutralization or both as its free base form and as its acid addition salt form due to incomplete neutralization,
the metal salt is at least one metal salt selected from the group consisting of sodium chloride, magnesium chloride, potassium chloride, sodium citrate, sodium tartrate, sodium bromide and sodium succinate, and
the content of the metal salt is the same or less number of moles of the acid in the basic drug acid addition salt;
the adsorbent is an adsorbent that adsorbs a polar solvent comprised in the patch wherein the adsorbent is at least one adsorbent selected from the group consisting of talc, kaoline, bentonite, hydrous silica, fumed silica, aminoalkyl methacrylate copolymer, crospovidone, carboxy vinyl polymer, zinc oxide, dextrin and dried aluminum hydroxide gel;
the pressure-sensitive adhesive base is selected from acrylic pressure-sensitive adhesive bases, rubber pressure-sensitive adhesive bases and silicone pressure-sensitive adhesive bases; and
wherein the basic drug acid addition salt is selected from any one from the group of flurazepam hydrochloride, rilmazafone hydrochloride, medetomidine hydrochloride and dexmedetomidine hydrochloride, butorphanol tartrate, perisoxal citrate, methamphetamine hydrochloride, methylphenidate hydrochloride, imipramine hydrochloride, sertraline hydrochloride, paroxetine hydrochloride, citalopram hydrobromide, fluoxetine hydrochloride, chlorpromazine hydrochloride, lidocaine hydrochloride, procaine hydrochloride, tetracaine hydrochloride, dibucaine hydrochloride, propitocaine hydrochloride, propiverine hydrochloride, solifenacin succinate, tizanidine hydrochloride, eperisone hydrochloride, ritodrine hydrochloride, meluadrine tartrate, trihexyphenidyl hydrochloride, amantadine hydrochloride, talipexole hydrochloride, selegiline hydrochloride, loberine hydrochloride, naloxone hydrochloride, ergotamine tartrate, flunarizine hydrochloride, cyproheptadine hydrochloride, diphenylpyraline hydrochloride, tulobuterol hydrochloride, procaterol hydrochloride, clenbuterol hydrochloride, fenoterol hydrobromide, isoprenaline hydrochloride, dopamine hydrochloride, diltiazem hydrochloride, verapamil hydrochloride, nicametate citrate, tolazoline hydrochloride; varenicline tartrate, flunarizine hydrochloride, nicardipine hydrochloride, manidipine hydrochloride, benidipine hydrochloride, temocapril hydrochloride, imidapril hydrochloride, metoprolol tartrate, betaxolol hydrochloride, arotinolol hydrochloride, celiprolol hydrochloride, carteolol hydrochloride, bevantolol hydrochloride, clonidine hydrochloride, propranolol hydrochloride, alprenolol hydrochloride, procainamide hydrochloride, mexitilene hydrochloride, procarbazine hydrochloride, irinotecan hydrochloride, buformin hydrochloride, cetraxate hydrochloride, azelastine hydrochloride, difenidol hydrochloride, bacampicillin hydrochloride, ticlopidine hydrochloride, galantamine hydrobromide, ondansetron hydrochloride, granisetron hydrochloride, ramosetron hydrochloride, azasetron hydrochloride, morphine hydrochloride, cocaine hydrochloride, pethidine hydrochloride, terbinafine hydrochloride, butenafine hydrochloride, amorolfine hydrochloride, and neticonazole hydrochloride,
wherein the neutralizer is sodium hydroxide, potassium hydroxide or magnesium hydroxide.

## Patentansprüche

1. Pflaster, umfassend eine druckempfindliche Klebeschicht, eine Trägerschicht und eine Trennschicht, wobei die druckempfindliche Klebeschicht auf die Trägerschicht laminiert ist und die Trennschicht auf der druckempfindlichen Klebeschicht befestigt ist, wobei die druckempfindliche Klebeschicht ein basisches Medikament, ein Metallsalz, ein Adsorptionsmittel und eine druckempfindliche Klebstoffgrundlage umfasst, wobei:
wobei das basische Medikament in seiner freien Basenform und das Metallsalz durch eine Neutralisationsreaktion zwischen dem Medikamentsäureadditionssalz und einem Neutralisationsmittel erhältlich ist, das in einer Menge zugesetzt wird, um das basische Medikament ganz oder teilweise in den Zustand des freien basischen Medikaments zu überführen,
das basische Medikament in der druckempfindlichen Klebeschicht als seine freie Basenform durch Neutralisation oder sowohl als seine freie Basenform als auch als seine Säureadditionssalzform durch unvollständige Neutralisation vorliegt,
das Metallsalz mindestens ein Metallsalz ist, ausgewählt aus der Gruppe bestehend aus Natriumchlorid, Magnesiumchlorid, Kaliumchlorid, Natriumcitrat, Natriumtartrat, Natriumbromid und Natriumsuccinat, und
der Gehalt des Metallsalzes die gleiche oder eine geringere Anzahl von Molen der Säure im basischen Medikamentadditionssalz ist;
das Adsorptionsmittel ein Adsorptionsmittel ist, das ein in dem Pflaster enthaltenes polares Lösungsmittel adsorbiert, worin das Adsorptionsmittel mindestens ein Adsorptionsmittel ist, ausgewählt aus der Gruppe bestehend aus Talkum, Kaolin, Bentonit, wasserhaltigem Siliziumdioxid, pyrogenem Siliziumdioxid, Aminoalkylmethacrylat-Copolymer, Crospovidon, Carboxyvinylpolymer, Zinkoxid, Dextrin und getrocknetem Aluminiumhydroxidgel;
die druckempfindliche Klebstoffgrundlage ausgewählt ist aus druckempfindlichen Acrylklebstoffgrundlagen, druckempfindlichen Gummiklebstoffgrundlagen und druckempfindlichen Silikonklebstoffgrundlagen; und
wobei das basische Medikamentsäureadditionssalz ausgewählt ist aus einer Gruppe von Flurazepamhydrochlorid, Rilmazafonhydrochlorid, Medetomidinhydrochlorid und Dexmedetomidinhydrochlorid, Butorphanoltartrat, Perisoxalcitrat, Methamphetaminhydrochlorid, Methylphenidathydrochlorid, Imipraminhydrochlorid, Sertralinhydrochlorid, Paroxetinhydrochlorid, Citalopramhydrobromid, Fluoxetinhydrochlorid, Chlorpromazinhydrochlorid, Lidocainhydrochlorid, Procainhydrochlorid, Tetracainhydrochlorid, Dibucainhydrochlorid, Propitocainhydrochlorid, Propiverinhydrochlorid, Solifenacinsuccinat, Tizanidinhydrochlorid, Eperisonhydrochlorid, Ritodrinhydrochlorid, Meluadrintartrat, Trihexyphenidylhydrochlorid, Amantadinhydrochlorid, Talipexolhydrochlorid, Selegilinhydrochlorid, Loberinhydrochlorid, Naloxonhydrochlorid, Ergotamintartrat, Flunarizinhydrochlorid, Cyproheptadinhydrochlorid, Diphenylpyralinhydrochlorid, Tulobuterolhydrochlorid, Prokaterolhydrochlorid, Clenbuterolhydrochlorid, Fenoterolhydrobromid, Isoprenalinhydrochlorid, Dopaminhydrochlorid, Diltiazemhydrochlorid, Verapamilhydrochlorid, Nicametatcitrat, Tolazolinhydrochlorid; Vareniclin-Tartrat, Flunarizinhydrochlorid, Nikardipinhydrochlorid, Manidipinhydrochlorid, Benidipinhydrochlorid, Temocaprilhydrochlorid, Imidaprilhydrochlorid, Metoprololtartrat, Betaxololhydrochlorid, Arotinololhydrochlorid, Celiprololhydrochlorid, Carteolhydrochlorid, Bevantololhydrochlorid, Clonidinhydrochlorid, Propranolhydrochlorid, Alprenolhydrochlorid, Procainamidhydrochlorid, Mexitilinhydrochlorid, Procarbazinhydrochlorid, Irinotecanhydrochlorid, Buforminhydrochlorid, Cetraxathydrochlorid, Azelastinhydrochlorid, Difenidolhydrochlorid, Bacampicillinhydrochlorid, Ticlopidinhydrochlorid, Galantaminhydrobromid, Ondansetronhydrochlorid, Granisetronhydrochlorid, Ramosetronhydrochlorid, Azasetronhydrochlorid, Morphinhydrochlorid, Kokainhydrochlorid, Pethidinhydrochlorid, Terbinafinhydrochlorid, Butenafinhydrochlorid, Amorolfinhydrochlorid und Neticonazolhydrochlorid,
wobei das Neutralisationsmittel Natriumhydroxid, Kaliumhydroxid oder Magnesiumhydroxid ist.

## Revendications

1. Timbre comprenant une couche adhésive sensible à la pression, une couche dorsale et une feuille de décollement, la couche adhésive sensible à la pression étant stratifiée sur la couche dorsale et la feuille de décollement étant fixée sur la couche adhésive sensible à la pression, ladite couche adhésive sensible à la pression comprenant un médicament de base, un sel métallique, un agent adsorbant et une base adhésive sensible à la pression, où :
le médicament de base dans sa forme de base libre et le sel métallique pouvant être obtenus par une réaction de neutralisation entre le sel d'addition acide médicament et un agent de neutralisation qui est ajouté en une quantité permettant de convertir le médicament de base en totalité ou en partie sous l'état de médicament en base libre,
le médicament de base est présent dans la couche adhésive sensible à la pression sous sa forme de base libre par neutralisation ou les deux sous sa forme de base libre et comme sa forme de sel d'addition acide due à la neutralisation incomplète,
le sel métallique est au moins un sel métallique sélectionné dans le groupe constitué du chlorure de sodium, du chlorure de magnésium, du chlorure de potassium, du citrate de sodium, du tartrate de sodium, du bromure de sodium et du succinate de sodium, et
la teneur du sel métallique est le même nombre ou un nombre inférieur de moles de l'acide dans le sel d'addition acide médicament de base ;
l'agent adsorbant est un agent adsorbant qui adsorbe un solvant polaire compris dans le timbre où l'agent adsorbant est au moins un agent adsorbant sélectionné dans le groupe constitué du talc, du kaolin, de la bentonite, de la silice hydratée, de la silice sublimée, du copolymère de méthacrylate d'aminoalkyle, de la crospovidone, du polymère carboxy vinylique, de l'oxyde de zinc, de la dextrine et du gel d'hydroxyde d'aluminium sec ;
la base adhésive sensible à la pression est sélectionnée parmi les bases adhésives acryliques sensibles à la pression, les bases adhésives de caoutchouc sensibles à la pression et les bases adhésives de silicone sensibles à la pression ; et
où le sel d'addition acide médicament de base est sélectionné parmi l'un quelconque du groupe du chlorhydrate de flurazépam, du chlorhydrate de rilmazafone, du chlorhydrate de médétomidine et du chlorhydrate de dexmédétomidine, du tartrate de butorphanol, du citrate de périsoxal, du chlorhydrate de méthamphétamine, du chlorhydrate de méthylphénidate, du chlorhydrate d'imipramine, du chlorhydrate de sertraline, du chlorhydrate de paroxétine, du bromhydrate de citalopram, du chlorhydrate de fluoxétine, du chlorhydrate de chlorpromazine, du chlorhydrate de lidocaïne, du chlorhydrate de procaïne, du chlorhydrate de tétracaïne, du chlorhydrate de dibucaïne, du chlorhydrate de propitocaïne, du chlorhydrate de propivérine, du succinate de solifénacine, du chlorhydrate de tizanidine, du chlorhydrate d'épérisone, du chlorhydrate de ritodrine, du tartrate de méluadrine, du chlorhydrate de trihexyphénidyle, du chlorhydrate d'amantadine, du chlorhydrate de talipexole, du chlorhydrate de sélégiline, du chlorhydrate de loberine, du chlorhydrate de naloxone, du tartrate d'ergotamine, du chlorhydrate de flunarizine, du chlorhydrate de cyproheptadine, du chlorhydrate de diphénylpyraline, du chlorhydrate de tulobutérol, du chlorhydrate de procatérol, du chlorhydrate de clenbutérol, du bromhydrate de fénotérol, du chlorhydrate d'isoprénaline, du chlorhydrate de dopamine, du chlorhydrate de diltiazem, du chlorhydrate de vérapamil, du citrate de nicamétate, du chlorhydrate de tolazoline, du tartrate de varénicline, du chlorhydrate de flunarizine, du chlorhydrate de nicardipine, du chlorhydrate de manidipine, du chlorhydrate de bénidipine, du chlorhydrate de témocapril, du chlorhydrate d'imidapril, du tartrate de métoprolol, du chlorhydrate de bétaxolol, du chlorhydrate d'arotinolol, du chlorhydrate de céliprolol, du chlorhydrate de cartéolol, du chlorhydrate de bévantolol, du chlorhydrate de clonidine, du chlorhydrate de propranolol, du chlorhydrate d'alprénolol, du chlorhydrate de procaïnamide, du chlorhydrate de mexitilène, du chlorhydrate de procarbazine, du chlorhydrate d'irinotécan, du chlorhydrate de buformine, du chlorhydrate de cétraxate, du chlorhydrate d'azélastine, du chlorhydrate de difénidol, du chlorhydrate de bacampicilline, du chlorhydrate de ticlopidine, du bromhydrate de galantamine, du chlorhydrate d'ondansétron, du chlorhydrate de granisétron, du chlorhydrate de ramosétron, du chlorhydrate d'azasétron, du chlorhydrate de morphine, du chlorhydrate de cocaïne, du chlorhydrate de péthidine, du chlorhydrate de terbinafine, du chlorhydrate de buténafine, du chlorhydrate d'amorolfine, et du chlorhydrate de néticonazole,
où l'agent de neutralisation est l'hydroxyde de sodium, l'hydroxyde de potassium ou l'hydroxyde de magnésium.
